# EUROPÄISCHE PATENTSCHRIFT

(11) **EP 3 024 811 B1**
(45) Veröffentlichungstag und Bekanntmachung des Hinweises auf die Patenterteilung: **20.11.2019**
(21) Anmeldenummer: 14739424.1
(22) Anmeldetag: 15.07.2014
(51) Int. Cl.: C07C 45/75, C07C 45/82, C07C 47/22, F23G 5/14, F23G 7/00, F23G 7/06

(54) **KREISLAUFFÜHRUNG UND ENTSORGUNG DER WÄSSRIGEN KATALYSATORLÖSUNG BEI AMINKATALYTISCHEN PROZESSEN**
CIRCULATION AND DISPOSAL OF THE AQUEOUS CATALYST SOLUTION IN AMINE CATALYTIC PROCESSES
CIRCULATION ET ÉLIMINATION DE LA SOLUTION AQUEUSE DE CATALYSEUR DANS DES PROCESSUS D'AMINOCATALYSE

(30) Priorität: 24.07.2013 EP 13177840
(43) Veröffentlichungstag der Anmeldung: 01.06.2016
(73) Patentinhaber: Röhm GmbH, 64293 Darmstadt (DE)
(72) Erfinder: KRILL, Steffen, 64367 Mühltal (DE); BALDUF, Torsten, 64319 Pfungstadt (DE); BURGHARDT, Rudolf, 64287 Darmstadt (DE); KÖLBL, Gerhard, 64579 Gernsheim (DE); ZHAO, Reed, Shanghai (CN)
(74) Vertreter: Röhm Patent Association
(86) Internationale Anmeldenummer: PCT/EP2014/065067
(87) Internationale Veröffentlichungsnummer: WO 2015/010944

(56) Entgegenhaltungen:
- DE-A1- 3 004 186
- DE-A1- 3 213 681

## Beschreibung

Die vorliegende Erfindung betrifft die oxidative Verbrennung von aminhaltigen Abwässern, insbesondere in einem Verfahren zur Herstellung von Methacrolein.

Methacrolein wird in der chemischen Synthese insbesondere als Zwischenprodukt zur Herstellung von Methacrylsäure, Methylmethacrylat oder auch von Wirk-, Geruchs- oder Geschmacksstoffen verwendet. Insbesondere betrifft die vorliegende Erfindung eine oxidative Verbrennung der aminhaltigen Abwässer unter nur geringer Stickoxidbildung.

Es besteht ein großes Interesse an möglichst einfachen, wirtschaftlichen und umweltschonenden Herstellungsverfahren für Methacrolein.

### Stand der Technik

Es sind diverse aminkatalytische Prozesse bekannt, bei denen aminhaltiges Reaktionswasser anfällt. Ein Beispiel dafür ist das großtechnisch bedeutende Verfahren zur Herstellung von Methacrolein, ausgehend von Propanal und Formaldehyd. Die Reaktion erfolgt über eine Mannich-Reaktion. Ein solches Verfahren zur Herstellung von Methacrolein, ist unter anderem in den Druckschriften US 7,141,702, US 4,408,079, JP 3069420, JP 4173757, EP 0 317 909 und US 2,848,499 beschrieben.

Von besonderem Interesse ist es dabei naturgemäß, diese Reaktion kontinuierlich durchzuführen. Dabei fällt als Nebenprodukt der Mannich-Reaktion oder einer Aldol-ähnlichen-Addition aminhaltiges Wasser an. Dieses Wasser stellt einerseits das Reaktionsmedium und Lösungsmittel für Edukte und Katalysatorlösung dar, und wird insbesondere zur Moderierung der Reaktionswärme und der Reaktionsführung benötigt. Auf der anderen Seite wird in einem solchen Prozess das Wasser, z.B. den Katalysator enthaltend, zurückgeführt. Ein Nachteil dieses Vorgehens ist jedoch, dass sich mit der Zeit das Wasser in dem Reaktionskreislauf anreichert und entfernt werden muss. Nach der weitgehenden Entfernung vom Reaktionsprodukt, dem Methacrolein, wird das Wasser zusammen mit aktiven und inaktiven Katalysatorbestandteilen zur Entsorgung in Form einer oxidativen Verbrennung einem Thermal Oxidizer zugeführt. Zu berücksichtigen ist weiter, dass auch mit dem Ausgangsmaterial Formalin, erhebliche Mengen Wasser ins System gelangen, je nach gewählter Stärke bzw. Konzentration des Formalins, üblicherweise werden handels- und produktionsübliche Formalinkonzentrationen zwischen 36 - 60 wt% Formaldehyd in Wasser eingesetzt, ergibt sich so per se eine Grundlast an Wasser, die nach Abtrennung vom Wertprodukt Methacrolein zusammen mit dem während der Reaktion gebildeten Wasser behandelt werden muss. Weiterhin muss bezüglich dem Wasserhaushalt in Reaktion und Aufarbeitung berücksichtigt werden, dass auch die Nebenreaktionen Wasser freisetzen. Dies gilt sowohl für die Nebenreaktionen des Propanals als auch für die Nebenprodukte der katalytisch aktiven Amine. Die katalytisch aktiven Amine bilden unter den Reaktionsbedingungen nach Eschweiler-Clarke bzw. in einer Leukart Wallach ähnlichen Reaktion höher-alkylierte Derivate, die teilweise nicht mehr katalytisch aktiv sind. So bildet sich beispielhaft aus Dimethylamin durch Reaktion mit einem Formaldehyd Equivalent Trimethylamin, während Wasser freigesetzt wird.

In DE 32 13 681 werden diesbezüglich mehrere Alternativen vorgeschlagen. Bei geringen Katalysatormengen in der wässrigen Phase, bzw. dem Destillationssumpf, der neben der wässrigen Phase und dem Katalysator zusätzlich hochsiedende Nebenprodukte und restliche Edukte und Methacrolein enthält, wird vorgeschlagen diesen zu entsorgen. Bei höheren Konzentrationen wird vorgeschlagen, den Sumpf einer weiteren, sehr aufwendigen Destillation zu unterziehen, um die Wassermenge zu reduzieren. In diesem Falle wird der Rest dann zurück in den Reaktionsraum geführt. Dieses Vorgehen ist jedoch nicht nur energetisch ungünstig, sondern es wird bei einer solchen weiteren Destillation zugleich ausbeutesenkend Methacrolein verworfen, welches aufgrund des geringeren Siedepunktes mit abdestilliert wird.

In einer dritten Alternative der DE 32 13 681 wird der Sumpf geteilt und ein Teil derart in den Reaktionsraum zurückgeführt, dass der Wassergehalt dort konstant bleibt. Der andere Teil des Sumpfes - und damit die wässrigen Phase - wird verworfen. DE 32 13 681 offenbart dabei nicht, ob und wie diese wässrige Lösung aufgearbeitet wird.

Wenn das Wasser nicht abdestilliert wird, kann dieses aufgrund einer relativ hohen Belastung mit Katalysator, Edukten, Produkt und Nebenprodukten in der Regel keiner biologischen Verwertung zugeführt werden. Stattdessen muss das Abwasser einer oxidativen Verbrennung unterzogen werden. Aufgrund des hohen Amingehalts z.B. im Abwasser einer Mannich-Reaktion oder einer Aldolkondensation entstehen bei einer solchen Verbrennung nach Stand der Technik große Mengen Stickoxide. Diese müssen im Weiteren katalytisch zersetzt oder abgetrennt werden, bevor die Verbrennungsgase emittiert werden können. Ein solches Vorgehen ist mit entsprechenden Investitionskosten verbunden.

Eine einfache bekannte Alternative stellt das Zuspeisen von Ammoniak in den Thermal Oxidizer dar. Ein solcher Thermal Oxidizer lässt sich grob in drei Bereiche gliedern. Im ersten Bereich befindet sich der Brenner. An dieser Stelle werden die zu verbrennende Flüssigkeit, in diesem Fall eine aminhaltige wässrige Lösung, und Luft zugespeist. Im darauf folgenden zweiten Bereich erfolgt eine oxidative Verbrennung. Aus Aminen werden in dieser Zone Stickoxide gebildet. In einer dritten Zone, am Ende des Thermal Oxidizers ist der Sauerstoffgehalt derart gering, dass die Verbrennung eher reduktiv erfolgt. Man kann die zweite Stufe entsprechend auch als oxidative Zone und den dritten Bereich als reduktive Zone des Thermal Oxidizers bezeichnen. In der reduktiven Zone ist der Sauerstoffgehalt in der Regel bei unter 2,5 Vol%.

Aus dem Stand der Technik ist es nun bekannt, dass man den Stickoxidanteil vor der letztendlichen Emission durch Zuleiten von Ammoniak in die reduktive Zone reduzieren kann. Nachteil dieses Vorgehens ist es jedoch, dass man neben der Lufteinspeisung eine weitere Zuleitung braucht und eine äquimolare Menge Ammoniak einsetzen muss, um die Stickoxidbildung zu reduzieren bzw. gebildete Stickoxide wieder zu reduzieren, um die national gültigen, genehmigungsrechtlichen Vorgaben zu erfüllen.

Es besteht also der dringende Bedarf an einer effektiven und gleichzeitig umweltschonenden Methode zur Verbrennung von aminhaltigen Reaktionswässern bei der Herstellung von Methacrolein nach dem oben erläuterten Verfahren.

### Aufgabe

In Anbetracht des Standes der Technik war es daher Aufgabe der vorliegenden Erfindung, das in einer aminkatalytischen Reaktion anfallende aminhaltige Abwasser unter Vermeidung der Bildung von Stickoxiden über eine Abluftkonzentration von 500 ppm, insbesondere von 250 ppm hinaus zu entsorgen.

In Anbetracht des Standes der Technik war es insbesondere Aufgabe, ein Verfahren zur Verfügung zu stellen, mit dem es möglich ist das aminhaltige Abwasser unter Vermeidung der Bildung von Stickoxiden über die bestehende Grundlast eines Thermal Oxidizers hinaus zu entsorgen.

Zusätzlich war es Aufgabe der vorliegenden Erfindung, dabei möglichst wenig Ammoniak einzusetzen.

Insbesondere war es Aufgabe der vorliegenden Erfindung, das aminhaltige Reaktionswasser einer kontinuierlich betriebenen Mannich-Reaktion zur Herstellung von Methacrolein umweltschonend zu entsorgen.

Ferner sollte das Verfahren mit relativ einfachen und kostengünstigen Modifikationen bestehender Anlagen realisiert werden können. Die Modifikationen sollten demgemäß mit geringen Investitionskosten verbunden sein. Hierbei sollten die Anlagen auch nach der Modifikation einfach zu warten sein und geringe Unterhaltskosten verursachen.

Weitere nicht explizit genannte Aufgaben ergeben sich aus dem Gesamtzusammenhang der nachfolgenden Beschreibung und der Ansprüche.

### Lösung

Gelöst werden diese Aufgaben durch ein neuartiges Verfahren zur oxidativen Verbrennung von aminhaltigem Reaktionswasser. In diesem Verfahren wird das aminhaltige Reaktionswasser in einem Thermal Oxidizer verbrannt. Ein solcher Thermal Oxidizer besteht in der Regel aus einer ersten Zone, aufweisend den Brenner, die Brennstoffzufuhr und die Luftzufuhr, einer zweiten oxidativen Zone und einer dritten, reduktiven Zone mit einem Sauerstoffgehalt kleiner 3 Vol%.

Das Verfahren kann somit z.B. unter einer leichten Modifikation bekannter Thermal Oxidizer erfolgen. Dies erfolgt in Form einer Ergänzung einer Zuleitung in die erste, die zweite, oxidative oder die dritte, reduktive Zone des Thermal Oxidizers.

Die Erfindung, für die vorliegend Schutz beansprucht wird, besteht aus einem Teil der im Folgenden beschriebenen Offenbarung, nämlich Verfahren zur Entsorgung von aminhaltigen Reaktionswässern wie in Anspruch 1 definiert.

Die erfindungsgemäß eingesetzten Thermal Oxidizer verfügen über mindestens drei Zonen. Bei der ersten handelt es sich um den Brenner, umfassend eine Brennkammer. Bei vielen Anlagen ist diese Brennkammer von der weiterführenden zweiten Zone räumlich, mit Überleitung abgetrennt, so dass die Differenzierung beider Zonen für den Fachmann leicht ersichtlich ist. In Ausführungsformen, bei denen beide Zonen nahtlos in einander übergehen, ist die Grenze zur zweiten Zone dort zu sehen, wo die Temperatur unterhalb von 1100 °C liegt. In der Flamme selbst wird diese Temperatur zumeist nicht unterschritten. Die erste Zone, auch Brennkammer oder Burner genannt, umfasst neben dem, zumeist mit Erdgas betriebenen Brenner, weiterhin eine Luftzufuhr und optional eine Zuleitung für flüssige Brennstoffe. Bei diesen flüssigen Brennstoffen kann es sich um organische Verbindungen, wie zum Beispiel Schweröl oder Lösungsmittel, oder um organische, leicht brennbare Abfälle einer Anlage handeln. Die flüssigen Brennstoffe werden zum einen zur zusätzlichen Befeuerung benötigt und zum anderen können hier organische, nicht aminhaltige Nebenprodukte eines anderen Produktionsprozesses verbrannt und damit umweltgerecht entsorgt werden. Die zugeführte Luft kann vor der Zuleitung erhitzt werden, um so eine effizientere Verbrennung in diesem Bereich zu ermöglichen. Weiterhin optional können in der ersten Zone auch Ablüfte aus Produktionsprozessen, insbesondere solche, die organische Bestandteile enthalten zur zusätzlichen Verbrennung und/oder Erhöhung der Brennleistung zugeleitet werden.

In einer Ausführungsform wird das aminhaltige Reaktionswasser in diese erste Zone, in diesem Fall bevorzugt direkt in die Flamme des Brenners eingespeist.

In der erfindungsgemäßen zweiten Ausführungsform des Verfahrens wird das aminhaltige Reaktionswasser in die zweite, oxidative Zone eingespeist. Die zweite, oxidative Zone, oft als Combustion Chamber bezeichnet, zeichnet sich gegenüber der ersten Zone durch eine geringere Temperatur bei einem gleichzeitig hohen Sauerstoffgehalt aus. In der Regel ist der Sauerstoffgehalt im vorderen Bereich der zweiten Zone höher als im fortlaufenden Bereich. Die zweite Zone kann sowohl horizontal als auch vertikal aufgestellt sein. Die erste Zone ist bevorzugt vertikal aufgestellt. Bevorzugt wird die aminhaltige wässrige Lösung mittels einer Zweistoffdüse eingespeist. Mit einer solchen Zweistoffdüse wird eine optimale Zerstäubung und damit Umsetzung der organischen und aminhaltigen Anteile des eingedüsten Reaktionswassers erzielt. Neben dem Reaktionswasser wird über die Zweistoffdüse zur besseren Verteilung Luft, Dampf oder ein anderes Trägergas, bevorzugt Luft eingedüst. Zweistoffdüsen können auch in den anderen optionalen Ausführungsformen Verwendung finden.

Diese oxidative Zone des Thermal Oxidizers weist eine Temperatur zwischen 850 und 1100 °C, besonders bevorzugt zwischen 880 und 1080 °C und ganz besonders bevorzugt zwischen 900 und 950 °C auf. Der Sauerstoffgehalt liegt über die gesamte zweite, oxidative Zone betrachtet zwischen 5 und 21 Vol%, wobei ein Gefälle innerhalb der oxidativen Zone vorliegen kann. Bedingt durch die oxidativen Prozesse wird Sauerstoff in Richtung des Übergangs zur reduktiven Zone hin verbraucht. Entsprechend liegt der Sauerstoffgehalt im Bereich der Einspeisung bzw. Eindüsung des aminhaltgien Reaktionswassers bevorzugt zwischen 10 und 21 Vol%, besonders bevorzugt zwischen 15 und 21 Vol%.

In einer dritten Ausführungsform erfolgt das Eindüsen des aminhaltigen Reaktionswassers in eine dritte Zone des Thermal Oxidizers, in der eine reduktive Umsetzung erfolgt. Auch diese Zone ist zumeist räumlich, mit einem Durchlass von der zweiten Zone abgetrennt. Für Anlagen, in denen eine solche Abtrennung fehlt, ist der Übergang für den Fachmann über den Sauerstoffgehalt zu bestimmen. Die reduktive Zone zeichnet sich dadurch aus, dass der Sauerstoffgehalt unterhalb von 3 Vol% liegt. Die Temperatur innerhalb dieser dritten Zone ist zumeist der Temperatur in der zweiten Zone ähnlich und liegt in der Regel zwischen 850 und 1000 °C, insbesondere zwischen 900 und 950 °C. In dieser Zone kann auch - unabhängig von der jeweiligen Ausführungsform - die weiter unten beschriebene, optionale Eindüsung von Denitrifikationsreagenzien, z.B. wässrige Ammoniaklösung zur Reduzierung der Stickoxide erfolgen.

Unter Sauerstoffgehalt wird erfindungsgemäß der Gehalt an elementarem Sauerstoff verstanden. Chemisch mit anderen Elementen verbundener Sauerstoff, wie z.B. CO₂ oder CO, werden nicht zum Sauerstoffgehalt gezählt.

Auf diese dritte Zone folgend können erfindungsgemäß einsetzbare Thermal Oxidizer weitere Bestandteile aufweisen. Zu diesen weiteren Bestandteilen gehört beispielsweise eine bevorzugt vorhandene Verweilzone, in der kinetisch langsame reduktive Prozesse weiter erfolgen können. Daran können sich beispielsweise ein oder mehrere Wärmetauscher anschließen. Mittels dieser kann beispielsweise Dampf für die Produktionsanlagen, für die Zweistoffdüsen, zur Erwärmung der in der Brennkammer eingespeisten Luft oder zur Erwärmung der zu verbrennenden Abwasserströme erzeugt werden. Auf diese Weise kann eine energetisch effiziente Arbeitsweise in Bezug auf den eingesetzten Brennstoff des Thermal Oxidizers realisiert werden. Abschließend weist der Thermal Oxidizer einen Abluftschacht oder Vergleichbares auf.

Das erfindungsgemäße Verfahren wird zur Aufarbeitung von aminhaltigem Reaktionswasser aus einer - bevorzugt kontinuierlich durchgeführten - Mannich-Reaktion, bei der Propanal mit Formaldehyd zu Methacrolein umgesetzt wird, eingesetzt. Alternativ zur direkten Zugabe von Formaldehyd kann es sich dabei insbesondere auch um einen Prozess unter Zugabe von Formalin oder Paraformaldehyd, die im weiteren mit dem Begriff Formaldehyd explizit mit umfasst sind, handeln.

Die zur Herstellung von Methacrolein geeigneten, auf einer Mannich-Reaktion basierenden Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Insbesondere bezieht sich das erfindungsgemäß vor der Zuleitung des aminhaltigen Reaktionswassers besonders bevorzugt durchgeführte Verfahren auf eine kontinuierlich durchgeführte Mannich-Reaktion, wie sie in der europäischen Patentanmeldung mit dem Anmeldeaktenzeichen 13002076.1 offenbart ist. Erfindungsgemäß wird zur Verdeutlichung einer solchen bevorzugten Vorstufe auf den Offenbarungsgehalt dieser Anmeldung bezüglich der Methacrolein-Synthese Bezug genommen.

Eine solche, besonders bevorzugte Mannich-Reaktion wird in Gegenwart von 0,1 bis 20 Mol% organische Base, bevorzugt einem sekundären Amin, und 0,1 bis 20 Mol% Säure, jeweils bezogen auf das eingesetzte Propanal, bei einer Temperatur von 100 bis 300 °C und bei einem Druck von 5 bis 100 bar durchgeführt.

Bei den Säuren handelt es sich in der Regel um anorganische Säuren oder organische Mono-, Di- bzw. Polycarbonsäuren, bevorzugt Monocarbonsäuren, insbesondere aliphatische Monocarbonsäuren. Besonders bevorzugt wird zur Umsetzung von Propanal und Formaldehyd mindestens eine organische Säure, besonders bevorzugt Essigsäure eingesetzt. Der Anteil an Säure beträgt zwischen 0,1 und 20 Mol-%, vorteilhaft von 0,5 bis 10 Mol-%, bevorzugt 1 bis 5 Mol-%, bezogen auf Propanal.

Bei den organischen Basen handelt es sich um Amine, besonders bevorzugt um sekundäre Amine. Als Amine kommen beispielsweise in Betracht: Dimethylamin, Diethylamin, Methylethylamin, Methylpropylamin, Dipropylamin, Dibutylamin, Di-iso-propylamin, Di-iso-butylamin, Methyl-iso-propylamin, Methyl-iso-butylamin, Methyl- sek.-butylamin, Methyl-(2-methylpentyl)-amin, Methyl-(2-ethylhexyl)-amin, Pyrrolidin, Piperidin, Morpholin, N-Methylpiperazin, N-Hydroxyethylpiperazin, Piperazin, Hexamethylenimin, Diethanolamin, Methylethanolamin, Methylcyclohexylamin, Methylcyclopentalamin, Dicyclohexylamin oder entsprechende Gemische. Der Anteil an organischer Base beträgt zwischen 0,1 und 20, vorteilhaft von 0,5 bis 10, bevorzugt 1 bis 5 Mol-%, bezogen auf Propanal.

Das Verhältnis der Äquivalente Amin zu Säure wird vorzugsweise so gewählt, dass im Reaktionsgemisch vor der Reaktion ein pH-Wert von 2,5 bis 9 resultiert.

In dem aminhaltigen Reaktionswasser, das erfindungsgemäß entsorgt wird, können neben den genannten Komponenten und Nebenprodukten zusätzlich verbliebene Edukte oder deren Folgeprodukte enthalten sein. Insbesondere sind dabei die Katalysatorkomponenten, wie ein sekundäres Amin und eine organische Säure, sowie das daraus gebildete Salz zu nennen. Nebenprodukte dieser Katalysatoren sind bezüglich einer Entsorgung von besonderem Interesse. Hier sind als Beispiel vor allem höher alkylierte Amine, wie insbesondere Trimethylamin bei Verwendung von Dimethylamin als originärem Katalysatoramin aufzuführen. Auch kleine Mengen Edukte oder Produkt können in dem aminhaltigen Reaktionswasser enthalten sein. Beispiele hierfür sind Methacrolein, Formaldehyd, Paraformaldehyd und Propanal. Als Nebenprodukte der Reaktion, die gleichsam in dem aminhaltigen Reaktionswasser enthalten sind, wären beispielsweise Dimere, Oligomere oder Polymere des Methacrolein zu nennen. Weiterhin können je nach Prozessführung auch weitere Hilfsstoffe, wie organische Lösungsmittel, wie z.B. Methanol, Ameisensäure, Propanol, Dioxan, Tetrahydrofuran oder Methoxyethanol enthalten sein sowie weitere in der Reaktionsmatrize enthaltene oder entstehende Stoffe.

In einem solchen Verfahren erfolgt die Abtrennung des Produktes, also des Methacroleins, in der Regel über eine Destillationskolonne. Die Zuführung der aufzuarbeitenden Reaktionslösung kann dabei auf die Kolonne, in die Kolonne oder unterhalb der Kolonne erfolgen. Im Verdampferteil dieser Kolonne sammelt sich ein Sumpf, der überwiegend aus dem Reaktionswasser der Reaktion besteht bzw. die Wassermengen enthält, die mit der Formalinlösung in den Kreislaufprozess gelangen. Dieses enthält zusätzlich die Katalysatorkomponenten, wie zum Beispiel die organische Säure und das sekundäre Amin bzw. das aus diesen gebildete Salz, sowie Nebenprodukte der Reaktion, aus der Katalysatorlösung oder aus der Kombination aus beiden. Ein solches Nebenprodukt aus der Katalysatorlösung kann zum Beispiel Trimethylamin sein, das aus Dimethylamin gebildet wird. Diese wässrige Katalysatorlösung kann bevorzugt unterhalb des Zulaufs, insbesondere am Sumpf der Kolonne abgezogen werden. Ein so erzeugtes Reaktionswasser setzt sich dabei aus dem Wasser, welches als Katalysatorlösung zugegeben wurde, dem bei der Reaktion gebildeten und optional dem aus der Formaldehydlösung zusammen. Weitere, jedoch in geringerem Umfang zu berücksichtigende Wasserquellen sind Bestandteile der technischen Edukte wie Propional und Wasser, das in diversen Nebenreaktionen der Katalysatorkomponten mit Edukten, Nebenprodukten und Reaktionsprodukten gebildet wird, sowie Reaktionswasser aus allen diesen Komponenten, die unter den Reaktionsbedingungen entstehen.

In einer alternativen Verfahrensgestaltung wird das aminhaltige Reaktionswasser von dem Produkt über einen Phasentrenner getrennt und aus diesem wird das aminhaltige Reaktionswasser direkt oder nach weiterer Aufarbeitung dem Thermal Oxidizer ganz oder teilweise zugeführt. Die organische Phase wiederum wird dann einer Destillationskolonne zugeführt, über die das Methacrolein gewonnen wird. Der Sumpf dieser Destillationskolonne wiederum kann entweder zurück in den Phasentrenner, in den Reaktionsraum oder gleichfalls ganz oder teilweise in den Thermal Oxidizer geführt werden.

Für die Weiterverarbeitung kann, wenn sehr wenig Amin und/oder Säure eingesetzt wird und sich deshalb die Rückführung des Katalysators nicht lohnt, die Sumpfflüssigkeit verworfen werden. Aufgrund der dennoch hohen organischen Belastung, insbesondere mit Aminen steht ein solches Wasser keiner direkten biologischen Aufarbeitung zur Verfügung und muss thermisch, insbesondere über einen Thermal Oxidizer entsorgt werden.

Bei größeren Amin- und/oder Säurekonzentrationen im Sumpfablauf kann aber auch Wasser teilweise destillativ abgetrennt und die Katalysatorlösung wieder in den Reaktor zurückgeführt werden. Bei einem solchen Verfahren reichern sich jedoch Nebenprodukte in dieser Katalysatorlösung an, so dass diese regelmäßig erneuert und der ausgeschleuste Teil analog zu oben beschriebenen Verfahren thermisch entsorgt werden muss.

Außerdem ist es möglich, den Sumpfablauf so in zwei Teilströme aufzuteilen, dass ein Teilstrom gerade die Menge an Wasser mit sich führt, die bei der Reaktion gebildet bzw. mit den Ausgangsstoffen eingegeben wurde. Dieser Teilstrom wird dann ausgeschleust und der restliche Anteil in den Reaktor zurückgeführt. Der ausgeführte Teil würde bei diesem Vorgehen thermisch entsorgt.

Erfindungsgemäß besonders bevorzugt wird das aminhaltige Reaktionswasser teilweise unterhalb des Zulaufs, bevorzugt aus dem Sumpf einer Destillationskolonne entnommen und thermisch über einen beschriebenen Thermal Oxidizer entsorgt. Der Rest des Sumpfes wird in den Reaktionsraum der Anlage geleitet.

Bevorzugt wird das aminhaltige Reaktionswasser bezogen auf die Erdgasmenge des Brenners in einer Menge zwischen 0,3 und 3,6 mol/Nm³ in die oxidative Zone eindosiert. Weiterhin ist es bevorzugt, dass das eindosierte Amin bezogen auf das gesamte in den Thermal Oxidizer eindosierte Wasser in einer Konzentration zwischen 17 und 350 mol/m³ vorliegt.

In einer weiteren Ausführungsform der vorliegenden Erfindung wird das z.B. aus dem Sumpf der Destillationskolonne entnommene aminhaltige Reaktionswasser über eine Membrantrennstufe geleitet. Das dabei gewonnene Retentat, also die bezüglich Feedstrom vor die Membranstufe wasserärmere Phase kann dann entweder anteilig oder komplett in den Reaktionskreislauf zurückgeführt werden oder ausgeschleust und erfindungsgemäß dem Thermal Oxidizer zugeführt werden. Alternativ oder zusätzlich kann auch das Permeat, für den Fall, dass die organischen oder aminigen Bestandteile für eine anderweitige Verwertung bzw. Entsorgung zu hoch sind, erfindungsgemäß dem Thermal Oxidizer zugeführt werden. Auf diese Weise gelingt eine optimale Kontrolle von Wassergehalt und Katalysatorgehalt im Reaktionsraum, gleichzeitig eine optimale Ausnutzung und Reduzierung der für einen geforderten Umsatz notwendigen Katalystormenge und die sichere und umweltgerechte Entsorgung der verbleibenden wasserhaltigen Katalysatorlösung, wobei die im Thermal Oxidizer erzeugten Stickoxid Emissionen kontrolliert und reduziert werden können und gleichzeitig ein Brennwert in dieser dem Thermal Oxidizer zugeführten Phase erzeugt wird, der höher ist als derjenige, der ursprünglich per single path der Reaktion erzeugt wird. Hierdurch kann Erdgas bzw. allgemein Brennstoff eingespart werden.

Im erfindungsgemäßen Verfahrens wird zusätzlich in der reduktiven Zone des Thermal Oxidizers Ammoniak, Harnstoff oder ein Amin zudosiert. Diese Variante entspricht in Bezug auf dieses zusätzliche Merkmal dem Stand der Technik und dient dazu, den Anteil entstandener Stickoxide weiter zu reduzieren. Mehrheitlich wird dabei elementarer Stickstoff gebildet. Durch das erfindungsgemäße Verfahren ist es jedoch nur nötig, im Vergleich zum Stand der Technik deutlich reduzierten Mengen einer der aufgeführten Substanzen in die reduktive Zone einzuspeisen. Bevorzugt wird das Verfahren jedoch vollständig ohne diese zusätzliche Einspeisung durchgeführt.

In einer weiteren Variante des vorliegenden, erfindungsgemäßen Verfahrens wird in die erste oder zweite Zone des Thermal Oxidizers zusätzlich eine zu verbrennende Flüssigkeit, enthaltend organische Bestandteile, aus einem anderen Verfahren eindosiert. Diese Flüssigkeit kann dabei gleichfalls rein organisch oder als wässrige Lösung oder Mischung vorliegen. Die Eindosierung kann gewöhnlich direkt in die Flamme in der ersten Zone, gesondert in der zweiten Zone oder zusammen mit dem aminhaltigen Reaktionswasser erfolgen.

Besonders bevorzugt handelt es sich bei der zusätzlich in dieser Variante eindosierten Flüssigkeit um Abfallprodukte aus der Umsetzung von Methacrolein zu Methylmethacrylat und/oder der Umsetzung von Methacrolein direkt zur Methacrylsäure. Entsprechende Verfahren werden ausführlich bei Nagai et al., (http://www.sumitomo-chem.co.jp/english/rd/report/theses/docs/20040200_30a.pdf) beschrieben und diskutiert. Insbesondere finden sich hier die direkte oxidative Versesterung von Methacrolein zum MMA sowie die Gasphasenoxidation von Methacrolein zu Methacrylsäure.

In einer weiteren Variante des vorliegenden, erfindungsgemäßen Verfahrens wird in die erste oder zweite Zone des Thermal Oxidizers zusätzlich eine zu verbrennende Flüssigkeit bzw. zu verbrennende Gasströme, enthaltend organische Bestandteile, aus weiteren Verfahren eingespeist, insbesondere solchen, die die Edukte für das erfindungsgemäße Methacroleinverfahren zur Verfügung stellen. Hierbei sind vor allem Verfahren für die Herstellung der Ausgangstoffe des Methacrolein Prozesses zu nennen, wie das Propionalverfahren ausgehend von Ethylen und Synthesegas, sowie diverse Formalinverfahren ausgehend von Methanol. Diese Flüssigkeiten können dabei gleichfalls rein organisch oder als wässrige Lösung oder Mischung vorliegen. Die Eindosierung kann gewöhnlich direkt in die Flamme in der ersten Zone, gesondert in der zweiten Zone oder zusammen mit dem aminhaltigen Reaktionswasser erfolgen. Besonders bevorzugt handelt es sich bei der zusätzlich in dieser Variante eindosierten Flüssigkeit um Abfallprodukte aus der Umsetzung von Ethylen und Synthesegas zu Propanal und/oder der Umsetzung von Methanol zu Formalin.

Verfahren zur Herstellung von Propanal sind ebenfalls weitgehend in der Literatur beschrieben, sowie die Erzeugung von Pentenal-haltigen organischen Abfällen, die aus Nebenreaktion der Hydroformylierung von Ethylen durch Konsekutivreaktion des gewünschten Produkts entstehen und die nach dem erfindungsgemäßen Verfahren zusammen mit der aminhaltigen Phase des Methacrolein Prozesses mit verbrannt und thermisch verwertet werden können.

Ein großer Vorteil des erfindungsgemäßen Verfahrens ist, dass dieses mit relativ einfachen und kostengünstigen Anlagen durchgeführt werden kann. Die Anlagen sind mit geringen Investitionskosten verbunden. Hierbei sind die Anlagen einfach zu warten und verursachen geringe Unterhaltskosten.

Die nachfolgenden Beispiele dienen zur näheren Erläuterung bevorzugter Ausführungsformen der vorliegenden Erfindung, ohne dass hierdurch eine Begrenzung der Erfindung erfolgen soll.

### Beispiele

Es wurde ein Thermal Oxidizer, ausgelegt zur Verbrennung von 35 to Abwasser pro Stunde verwendet. Dieser erzeugt dabei 73 to Dampf (36 bar bei 343°C) pro Stunde. Dabei werden 4,5 to Erdgas pro Stunde verbrannt, was einer Leistungsaufnahme von ca. 62 MW entspricht. Der Thermal Oxidizer ist zusätzlich mit einer Zuleitung in die oxidative Zone des Thermal Oxidizers ausgerüstet. Diese weist eine luftbetriebene Zweistoffdüse auf. Als Reaktionswasser wurde ein Abwasser enthaltend 40 Gew% Dimethylamin verwendet. Die angegebenen Stickoxidkonzentrationen wurden im Abgas inline mittels IR-Messung gemessen und beziehen sich auf einen stabilen Zustand, der nach einigen Minuten Betrieb erreicht wird. Folgende Prozessparameter wurden bei allen Versuchen eingehalten: Flussrate des Abwassers: 21,6 bis 23,5 to / h
Temperatur Gasphase in reduktiver Zone: 870 bis 905 °C
Abgasmenge: 128 000 bis 132 000 m³ / h
Ablufzufuhr (zusätzliche Prozessluft mit geringem Organikanteil): 2000 Nm³ / h
Konzentration der Ammoniak-Lösung: 20 Gew%

In den Beispielen 1 bis 7 erfolgte die Zuleitung der aminhaltigen Reaktionswässer in die oxidative Zone des Thermal Oxidizers. In den erfindungsgemäßen Beispielen 1 bis 6 wurde zusätzlich eine Ammoniak-Lösung in die reduktive Zone des Thermal Oxidizers gesprüht. Im nicht erfindungsgemäßen Beispiel 7 wurde dieses unterlassen.

In den Grundlastbeispielen 1 bis 4 wurde auf die Zuleitung der Dimethylamin-Lösung verzichtet, in Grundlastbeispiel 1 zusätzlich auch auf die Zuleitung des Ammoniaks. Der in Grundlastbeispiel 1 gemessene Wert für die Stickoxidkonzentration im Abgas kann als Grundlast des Thermal Oxidizers betrachtet werden. Diese Stickoxide entstehen aus dem elementaren Stickstoff der zugeleiteten Luft, zumeist bereits in der Flamme.

In den nicht erfindungsgemäßen Beispielen 8 bis 10 erfolgte die Zuleitung der Dimethylamin-Lösung direkt in die erste Zone des Thermal Oxidizers (die Brennkammer).

| | Dimethylamin-Lösung | Ammoniak-Lösung | NOx Im Abgas |
|---|---|---|---|
| | kg/h | kg/h | mg/m³ |
| Beispiel 1 | 220 | 180 | 35 |
| Beispiel 2 | 400 | 180 | 35 |
| Beispiel 3 | 500-550 | 180 | 38 |
| Beispiel 4 | 600 | 150 | 37 |
| Beispiel 5 | 625 | 100 | 37 |
| Beispiel 6 | 650 | 50 | 40 |
| Beispiel 7 | 650-700 | 0 | 45-50 |
| Grundlastbeispiel 1 | 0 | 0 | 90-100 |
| Grundlastbeispiel 2 | 0 | 35 | 55 |
| Grundlastbeispiel 3 | 0 | 70 | 30 |
| Grundlastbeispiel 4 | 0 | 180 | 35 |
| Beispiel 8 | 550 | 70 | 40-45 |
| Beispiel 9 | 550 | 35 | 65-70 |
| Beispiel 10 | 550 | 0 | 155 |

Die Versuche zeigen, dass unabhängig von dem Einspeiseort es überraschend möglich ist, dass aminhaltige Reaktionswässer aus einem aminkatalytischem Prozess in einem Thermal Oxidizer unter Bildung von nur sehr geringen Mengen Stickoxid und unter Zuleitung von nur geringen Mengen oder gar keinem Ammoniak entsorgt werden können.

Der Vergleich von Beispiel 7 mit Beispiel 10 zeigt weiterhin, dass der Einspeiseort der aminhaltigen wässrigen Lösung in den Thermal Oxidizer einen zusätzlichen überraschenden Effekt in Bezug auf die Verminderung der Stickoxidbildung hat. Beispiel 7 zeigt darüber hinaus sehr überraschend, dass durch das erfindungsgemäße Verfahren auf die Ammoniak-Einspeisung in der reduktiven Zone des Thermal Oxidizers gänzlich verzichtet werden kann.

### Bezugszeichenliste

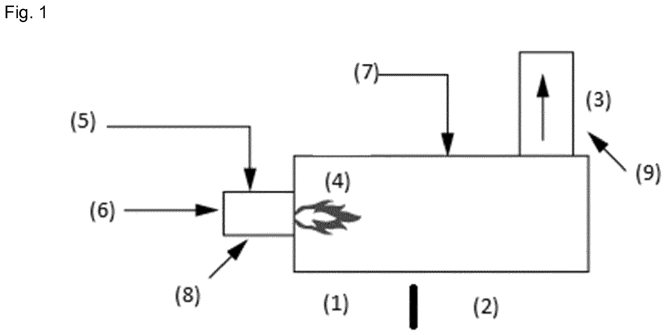

### Fig. 1: Erfindungsgemäß betriebener Thermal Oxidizer

- (1): Erste Zone des Thermal Oxidizers
- (2): Zweite, oxidative Zone des Thermal Oxidizers
- (3): Dritte, reduktive Zone des Thermal Oxidizers
- (4): Flamme
- (5): Luft- bzw. Sauerstoffzufuhr
- (6): Brennstoffzufuhr
- (7): Zufuhr des aminhaltigen Reaktionswassers
- (8): Optionale Zufuhr mindestens einer weiteren, zu verbrennenden Flüssigkeit oder mindestens eines zu verbrennenden Gases
- (9): Optionale Zufuhr von Ammoniak oder eines zweiten Amins

## Patentansprüche

1. Verfahren zur Entsorgung von aminhaltigen Reaktionswässern aus einem aminkatalytischen Prozess bei dem Propanal mit Formaldehyd, Formalin oder Paraformaldehyd zu Methacrolein umgesetzt wird, **dadurch gekennzeichnet, dass** die Entsorgung durch Verbrennung in einem Thermal Oxidizer erfolgt, wobei das aminhaltige Reaktionswasser in dem Thermal Oxidizer, bestehend aus einer ersten Zone, aufweisend den Brenner, die Brennstoffzufuhr und die Luftzufuhr, einer zweiten oxidativen Zone, in der eine Temperatur zwischen 850 und 1100 °C und ein Sauerstoffgehalt zwischen 5 und 21 Vol% vorliegt, und einer dritten, reduktiven Zone mit einem Sauerstoffgehalt kleiner 3 Vol% verbrannt wird, **dadurch gekennzeichnet, dass** der Übergang zwischen der ersten und der zweiten Zone an der Stelle des Thermal Oxidizers ist, an der die Temperatur im Thermal Oxidizer unter 1100 °C fällt, dass der Übergang zwischen der zweiten und der dritten Zone an der Stelle des Thermal Oxidizers ist, an der der Sauerstoffgehalt innerhalb des Thermal Oxidizers unter 3 Vol% fällt, dass das aminhaltige Reaktionswasser in die zweite oxidative Zone eingesprüht wird, und dass in der reduktiven Zone des Thermal Oxidizers zusätzlich Ammoniak, Harnstoff oder ein Amin zudosiert wird.

2. Verfahren gemäß Anspruch 1, **dadurch gekennzeichnet, dass** die Mannich-Reaktion in Gegenwart von 0,1 bis 20 Mol% Amin als organische Base, bevorzugt einem sekundären Amin, und 0,1 bis 20 Mol% Säure, jeweils bezogen auf Propanal, bei einer Temperatur von 100 bis 300 °C und bei einem Druck von 5 bis 100 bar kontinuierlich durchgeführt wird.

3. Verfahren gemäß Anspruch 1 oder 2, **dadurch gekennzeichnet, dass** das aminhaltige Reaktionswasser dem Sumpf einer Destillationskolonne entnommen wird und der Rest des Sumpfes in den Reaktionsraum der Anlage geleitet wird.

4. Verfahren gemäß mindestens einem der Ansprüche 1 bis 3, **dadurch gekennzeichnet, dass** es sich bei dem aminhaltigen Reaktionswasser um das Retentat des durch eine Membrantrennstufe geleiteten Sumpfes einer Destillationskolonne handelt.

5. Verfahren gemäß mindestens einem der Ansprüche 1 bis 4, **dadurch gekennzeichnet, dass** in die erste oder zweite Zone des Thermal Oxidizers zusätzlich mindestens eine zu verbrennende Flüssigkeit, enthaltend organische Bestandteile, aus einem anderen Verfahren eindosiert wird.

6. Verfahren gemäß Anspruch 5, **dadurch gekennzeichnet, dass** es sich bei dem anderen Verfahren um ein Verfahren zur Umsetzung von Methacrolein zu Methylmethacrylat oder Methacrylsäure handelt.

7. Verfahren gemäß Anspruch 5, **dadurch gekennzeichnet, dass** es sich bei dem anderen Verfahren um ein Verfahren zur Herstellung von Propionaldehyd ausgehend von Ethylen und Synthesegas handelt, bei dem eine die Nebenprodukte und Hochsieder enthaltende flüssige Abfallphase entsteht, die im Thermal Oxidizer verbrannt wird.

8. Verfahren gemäß mindestens einem der Ansprüche 1 bis 4, **dadurch gekennzeichnet, dass** in die erste oder zweite Zone des Thermal Oxidizers zusätzlich mindestens eine zu verbrennende gasförmige Phase, enthaltend organische Bestandteile und/oder Wasserstoff, aus einem anderen Verfahren eindosiert wird.

9. Verfahren gemäß Anspruch 8, **dadurch gekennzeichnet, dass** es sich bei dem anderen Verfahren um ein Verfahren zur Herstellung von Formalin ausgehend von Methanol handelt, bei dem ein wasserstoffhaltiges Abgas entsteht, das im Thermal Oxidizer verbrannt wird.

10. Verfahren gemäß mindestens einem der Ansprüche 1 bis 9, **dadurch gekennzeichnet, dass** das aminhaltige Reaktionswasser bezogen auf die Erdgasmenge des Brenners in einer Menge zwischen 0,3 und 3,6 mol/Nm³ in die oxidative Zone eindosiert wird.

## Claims

1. Method for disposing of amine-containing waters of reaction from an amine-catalytic process in which propanal is reacted with formaldehyde, formalin or paraformaldehyde to give methacrolein, **characterized in that** the disposal is effected by combustion in a thermal oxidizer,
wherein the amine-containing water of reaction is combusted in the thermal oxidizer consisting of a first zone including the burner, the fuel supply and air supply, a second, oxidative zone in which a temperature between 850 and 1100°C and an oxygen content between 5 and 21% by volume are present, and a third, reductive zone having an oxygen content less than 3% by volume, **characterized in that** the transition between the first and second zone is at the point in the thermal oxidizer where the temperature in the thermal oxidizer falls below 1100°C, **in that** the transition between the second and third zone is at the point in the thermal oxidizer where the oxygen content within the thermal oxidizer falls below 3% by volume, **in that** the amine-containing water of reaction is sprayed into the second, oxidative zone, and **in that** ammonia, urea or an amine is additionally metered into the reductive zone of the thermal oxidizer.

2. Method according to Claim 1, **characterized in that** the Mannich reaction is conducted continuously in the presence of 0.1 to 20 mol% of amine as organic base, preferably a secondary amine, and 0.1 to 20 mol% of acid, based in each case on propanal, at a temperature of 100 to 300°C and at a pressure of 5 to 100 bar.

3. Method according to Claim 1 or 2, **characterized in that** the amine-containing water of reaction is taken from the bottom of a distillation column and the rest of the bottoms is passed into the reaction space of the plant.

4. Method according to at least one of Claims 1 to 3, **characterized in that** the amine-containing water of reaction is the retentate from the bottoms of a distillation column which have been passed through a membrane separation stage.

5. Method according to at least one of Claims 1 to 4, **characterized in that** at least one liquid to be combusted, comprising organic constituents, from another process is additionally metered into the first or second zone of the thermal oxidizer.

6. Method according to Claim 5, **characterized in that** the other process is a process for converting methacrolein to methyl methacrylate or methacrylic acid.

7. Method according to Claim 5, **characterized in that** the other process is a process for preparing propionaldehyde proceeding from ethylene and synthesis gas, which gives rise to a liquid waste phase comprising the by-products and high boilers which is combusted in the thermal oxidizer.

8. Method according to at least one of Claims 1 to 4, **characterized in that** at least one gaseous phase to be combusted, comprising organic constituents and/or hydrogen, from another process is additionally metered into the first or second zone of the thermal oxidizer.

9. Method according to Claim 8, **characterized in that** the other process is a process for preparing formalin proceeding from methanol, which gives rise to a hydrogenous offgas which is combusted in the thermal oxidizer.

10. Method according to at least one of Claims 1 to 9, **characterized in that** the amine-containing water of reaction, based on the natural gas rate of the burner, is metered into the oxidative zone in an amount between 0.3 and 3.6 mol/m³ (STP).

## Revendications

1. Procédé d'élimination d'eaux de réaction contenant des amines issues d'un procédé aminocatalytique selon lequel du propanal est mis en réaction avec du formaldéhyde, de la formaline ou du paraformaldéhyde pour former de la méthacroléine, **caractérisé en ce que** l'élimination a lieu par combustion dans un oxydeur thermique, l'eau de réaction contenant des amines étant brûlée dans l'oxydeur thermique, constitué par une première zone, comprenant le brûleur, l'alimentation de combustible et l'alimentation d'air, une deuxième zone d'oxydation, dans laquelle une température comprise entre 850 et 1 100 °C et une teneur en oxygène comprise entre 5 et 21 % en volume sont présentes, et une troisième zone de réduction ayant une teneur en oxygène inférieure à 3 % en volume, **caractérisé en ce que** la transition entre la première et la deuxième zone se trouve à l'emplacement de l'oxydeur thermique auquel la température dans l'oxydeur thermique chute en dessous de 1 100 °C, et **en ce que** la transition entre la deuxième et la troisième zone se trouve à l'emplacement de l'oxydeur thermique auquel la teneur en oxygène dans l'oxydeur thermique chute en dessous de 3 % en volume, **en ce que** l'eau de réaction contenant des amines est injectée dans la deuxième zone d'oxydation, et **en ce que** de l'ammoniac, de l'urée ou une amine est en outre ajouté dans la zone de réduction de l'oxydeur thermique.

2. Procédé selon la revendication 1, **caractérisé en ce que** la réaction de Mannich est réalisée en continu en présence de 0,1 à 20 % en moles d'une amine en tant que base organique, de préférence d'une amine secondaire, et 0,1 à 20 % en moles d'un acide, à chaque fois par rapport au propanal, à une température de 100 à 300 °C et à une pression de 5 à 100 bar.

3. Procédé selon la revendication 1 ou 2, **caractérisé en ce que** l'eau de réaction contenant des amines est soutirée au fond d'une colonne de distillation et le reste du fond est acheminé dans la chambre de réaction de l'unité.

4. Procédé selon au moins l'une quelconque des revendications 1 à 3, **caractérisé en ce que** l'eau de réaction contenant des amines consiste en le rétentat du fond d'une colonne de distillation acheminé au travers d'une étape de séparation à membrane.

5. Procédé selon au moins l'une quelconque des revendications 1 à 4, **caractérisé en ce qu'**au moins un liquide à brûler, contenant des constituants organiques, est en outre introduit dans la première ou la deuxième zone de l'oxydeur thermique depuis un autre procédé.

6. Procédé selon la revendication 5, **caractérisé en ce que** l'autre procédé consiste en un procédé de transformation de méthacroléine en méthacrylate de méthyle ou acide méthacrylique.

7. Procédé selon la revendication 5, **caractérisé en ce que** l'autre procédé consiste en un procédé de fabrication de propionaldéhyde à partir d'éthylène et d'un gaz de synthèse, selon lequel une phase liquide de déchets contenant les produits secondaires et les composants de point d'ébullition élevé se forme, qui est brûlée dans l'oxydeur thermique.

8. Procédé selon au moins l'une quelconque des revendications 1 à 4, **caractérisé en ce qu'**au moins une phase gazeuse à brûler, contenant des constituants organiques et/ou de l'hydrogène, est en outre introduite dans la première ou la deuxième zone de l'oxydeur thermique à partir d'un autre procédé.

9. Procédé selon la revendication 8, **caractérisé en ce que** l'autre procédé consiste en un procédé de fabrication de formaline à partir de méthanol, selon lequel un gaz d'échappement contenant de l'hydrogène se forme, qui est brûlé dans l'oxydeur thermique.

10. Procédé selon au moins l'une quelconque des revendications 1 à 9, **caractérisé en ce que** l'eau de réaction contenant des amines est injectée dans la zone d'oxydation en une quantité comprise entre 0,3 et 3,6 mol/Nm³, par rapport à la quantité de gaz naturel du brûleur.
